# EUROPEAN PATENT SPECIFICATION

(11) **EP 0 512 844 B1**
(45) Date of publication and mention of the grant of the patent: **06.08.1997**
(21) Application number: 92304142.0
(22) Date of filing: 08.05.1992
(51) Int. Cl.: A61K 47/48

(54) **Targeted delivery of bone growth factors**
Osteogenische Wachstumsfaktoren zur Abgabe an Zielorgan
Délivrance ciblée de facteurs de croissance ostéogoniques

(30) Priority: 10.05.1991 US 698467
(43) Date of publication of application: 11.11.1992
(73) Proprietor: CELTRIX PHARMACEUTICALS, INC., Santa Clara, CA 95052-8203 (US)
(72) Inventor: Bentz, Hanne, Newark, California 9450 (US); Rosen, David, San Jose, California 95135 (US)
(74) Representative: Goldin, Douglas Michael

(56) References cited:
- WO-A-88/00837
- WO-A-90/05755
- STN FILE SERVER & FILE MEDLINE, AN=90272939; C.C. JOHNSTON. Jr.: "Treatment of osteoporotic patients"
- STN FILE SERVER & FILE MEDLINE, AN=92163668; C.H. TURNER: "Toward a cure for osteoporosis: reversal of excessive bone fragility"

## Description

### Technical Field

This invention relates to the fields of protein engineering and medical treatment. More particularly, this invention relates to conjugates of bone growth factors and targeting molecules and their uses in bone repair and augmentation and the like.

### Background of the Invention

"Transforming growth factor-β" (TGF-β) represents a family of proteins which are evolutionarily highly conserved, and which affect a broad spectrum of cell types. Two forms, TGF-β1 and TGF-β2, have been identified in platelet releasates and bone. Both proteins exist as 26 kD disulfide-linked homodimers. Even though there are 14 amino acid differences in the first 36 amino acids residues of the two forms, their biological activities are similar (Cheifetz et al., Cell (1987) 48:409-415; S. Seyedin et al., J. Biol. Chem. (1987) 262:1946-1949). Although TGF-β1 and TGF-β2 apparently bind to common cell surface receptors, the amino acid sequence homology is only about 70%. TGF-β3 exhibits about 76% homology with TGF-β1, and about 79% homology with TGF-β2.

Additional TGF-βs have also been described. U.S. Patent No. 4,886,747 describes the identification of TGF-β3 and its nucleotide sequence, and describes a method for recovery of TGF-β3 from recombinant cell cultures. S.B. Jakowlew et al., Molec. Endocrinol. (1988) 2:1186-1195 describes TGF-β4 and its nucleotide sequence, identified by cDNA characterization. A.B. Roberts et al., Growth Factors, Vol. 2 (1990), pp. 135-147, describes the purification of TGF-β5 from Xenopus-conditioned medium.

TGF-β was first identified as a factor which permitted anchorage-independent growth of primary cell cultures. In vivo, TGF-β promotes the deposition of connective tissue, and induces cell growth for cells of mesenchymal origin. TGF-β1 and TGF-β2 affect the proliferation and differentiation of cells of the immune system including macrophages (Wall et al., Proc. Natl. Acad. Sci. U.S.A. (1987) 84:5788), pre-B cells (Lee et al., J. Exp. Med. (1987) 166:1290), hematopoietic stem cells (Ohta et al., Nature (1987) 329:539; Ishibashi et al., Blood (1987) 69:1737; Keller et al., J. Exp. Med. (1988) in press; Sing et al., Blood (1988) in press), and NK cells (Rook et al., J. Immunol. (1986) 136:3916). Bentz et al., U.S. Patent No. 4,806,523, disclosed that TGF-β may also be administered to suppress inflammatory responses, including immune suppression. McPherson et al., U.S. Patent No. 4,816,442 disclosed that TGF-β may also be administered to suppress hyperproliferation, for example, cancer and leukemia.

Activins are dimeric proteins structurally similar to inhibin, TGF-β1, TGF-β2, and other proteins that makeup a family of proteins structurally related to TGF-β1. These proteins exhibit the chromatographic properties of TGF-βs. In addition to having homology with respect to the amino acid sequences, activins exhibit conservation of cysteine positions characteristic of the TGF-βs. Activins exhibit a molecular weight of 25 kD under nonreducing conditions by SDS-PAGE (and a molecular weight of 14 kD under reducing conditions). There are two known forms of the activin subunits, which have been termed βA or βB. Homodimeric forms βAA and βBB and a heterodimeric form βAB have been described in the literature. Activin subunits have about a 30% homology to TGF-β1 and TGF-β2 chains in terms of their amino acid sequences. Inhibins are polypeptides which are also structurally related to activins. Inhibins are heterodimers of the activin βA or βB subunit and a separate α subunit. Inhibins exhibit activity essentially opposite to activin.

The activin βA homodimer and βAB heterodimer have been purified from porcine follicular fluid, and have been shown to stimulate the release of follicle stimulating hormone (FSH) from rat pituitary cells *in vitro* (W. Vale et al., Nature (1986) 321:776-79). Other reported activities include stimulation of oxytocin release from neurosecretory neurons (P.E. Sawchemko, et al., Nature (1988) 334:615-17; W. Vale et al., "Recent Progress in Hormone Research" (1988) 44:1-34); stimulation of insulin secretion from pancreatic islets (Y. Totsuka et al., Biochem. & Biophys. Res. Comm. (1988) 156:335-39); and stimulation of erythroid and multipotential progenitor cell colony formation in bone marrow culture (J. Yu et al., Nature (1987) 330:765-67; H.E. Broxmeyer et al., Proc. Natl. Acad. Sci. U.S.A. (1988) 85:9052-56). Activin βA is apparently identical to erythroid differentiation factor (EDF) (M. Murata et al., Proc. Natl. Acad. Sci. U.S.A. (1988) 85:2434-38).

Despite the fact that activin is similar in amino acid sequence to TGF-β, activin does not compete with TGF-β for binding to TGF-β receptors types I, II, or III present on fibroblasts and epithelial cells. However, activin has been reported to compete against binding of TGF-β1 to rat pituitary tumor cells (S. Cheifetz et al., J. Biol. Chem. (1988) 263:17225-28). TGF-β1 and TGF-β2 have been reported to induce formation of endochondral bone in vivo (M.E. Joyce et al., J. Cell Biol. (1990) 110:2195-2207, H. Bentz, et al. (1989) J. Biol. Chem., 264:20805-10).

A "bone morphogenetic protein" (BMP) was extracted from demineralized bone using urea or guanidine hydrochloride and reprecipitated according to the disclosures in U.S. Patent Nos. 4,294,753 and 4,455,256 to Urist. Urist subsequently reported (Urist, M. R., Clin. Orthop. Rel. Res. (1982) 162:219) that ion exchange purification of this crude protein mixture yielded an activity which was unadsorbed to carboxymethyl cellulose resin (CMC) at pH 4.8. Urist's reports in Science (1983) 220:680-685 and Proc. Natl. Acad. Sci. U.S.A. (1984) 81:371-375 describe BMPs having molecular weights of 17,500 and 18,500 daltons. Urist's patent publication, EPA Publication No. 0212474, describes BMP fragments of 4,000 to 7,000 daltons obtained by limited proteolysis of BMP.

U.S. Patent No. 4,608,199 describes a bone-derived protein of 30,000-32,000 daltons. The protein is described as being water soluble and having no affinity for concanavalin A.

WO 88/00205 reports four proteins, designated BMP-1, BMP-2 Class I ("BMP-2"), BMP-3, and BMP-2 Class II ("BMP-4"), that are alleged to have osteogenic activity. It is not known whether these BMPs have osteogenic activity by themselves or require combination with other factors.

J.M. Wozney, in Growth Factor Research, Vol. 1 (1989), pp. 267-280, describes three additional BMP proteins closely related to BMP-2, and which have been designated BMP-5, BMP-6 and BMP-7.

WO 89/09787 and 89/09788 describe a protein called "OP-1", now known to be BMP-7. The cloning of BMP-7 is described in E. Ozkaynak et al., EMBO Journal (1990) 9:2085-2093, and the purification of BMP-7 is described in T.K. Sampath et al., J. Biol. Chem. (1990) 265:13198-13205.

U.S. Patent No. 4,434,094 to Seyedin and Thomas reported the partial purification of a bone generation-stimulating, bone-derived protein by extraction with chaotropic agents, fractionation on anion and cation exchange columns, and recovery of the activity from a fraction adsorbed to CMC at pH 4.8. This new protein fraction was termed "osteogenic factor" (OF) and was characterized as having a molecular weight below about 30,000 daltons.

There are several references in the art to proteins modified by covalent conjugation to polymers, to alter the solubility, antigenicity and biological clearance of the protein. For example, U.S. Patent No. 4,261,973 disclosed the conjugation of several allergens to PEG (polyethylene glycol) or PPG (polypropylene glycol) to reduce the proteins' immunogenicity. U.S. Patent No. 4,301,144 disclosed the conjugation of hemoglobin with PEG and other polymers to increase the protein's oxygen carrying capability. EPO 98,110 disclosed coupling an enzyme or interferon to a polyoxyethylene-polyoxypropylene (POE-POP) block polymer increases the protein's halflife in serum. U.S. Patent No. 4,179,337 disclosed conjugating hydrophilic enzymes and insulin to PEG or PPG to reduce immunogenicity. Davis et al., Lancet (1981) 2:281-83 disclosed the enzyme uricase modified by conjugation with PEG to provide uric acid metabolism in serum having a long halflife and low immunogenicity. Nishida et al., J. Pharm. Pharmacol. (1984) 36:354-55 disclosed PEG-uricase conjugates administered orally to chickens, demonstrating decreased serum levels of uric acid. Inada et al., Biochem. & Biophys. Res. Comm. (1984) 122:845-50 disclosed lipoprotein lipase conjugation with PEG to render it soluble in organic solvents. Takahashi et al., Biochem. & Biophys. Res. Comm. (1984) 121:261-65 disclosed HRP conjugated with PEG to render the enzyme soluble in benzene. Abuchowski et al., Cancer Biochem. Biophys. (1984) 7:175-86, disclosed that enzymes such as asparaginase, catalase, uricase, arginase, trypsin, superoxide dismutase, adenosine deaminase, phenylalanine ammonia-lyase, and the like, conjugated with PEG exhibit longer half-lives in serum and decreased immunogenicity. U.S. Patent No. 4,830,847, and European Patent Application Ser. No. 207,557 disclose diphosphonate-derivatized macromolecules which may be labeled with technium-99m. WO-A-90/05755 discloses conjugates of collagen cross-linked to a synthetic hydrophilic polymer to which factors aiding in healing or regrowth of normal tissue as for instance TGF may be chemically linked. The conjugates can augment tissues by direct application to the site requiring augmentation.

Both antibodies and liposomes have been used to direct drugs to target sites (see, for example, Tyle and Ram, eds., Targeted Therapeutic Systems, Marcel Dekker, New York, 1990.)

Targeted delivery of bone growth factors may reduce harmful or undesirable effects of those molecules, allow the use of lower doses because relatively higher doses can be delivered to the site of interest, and may prolong the effect at the site of interest. In addition, the use of a targeting molecule that influences bone metabolism, including bone resorption and formation, may result in an additive or synergistic effect.

### Summary of the Invention

It is an object of the invention to provide a composition comprising a bone growth factor and a targeting molecule having affinity for a tissue of interest, where the bone growth factor and targeting molecule are chemically conjugated to each other via a crosslinker. The crosslinker is preferably a synthetic hydrophilic polymer. The bone growth factor is preferably TGF-β, activin, or bone morphogenic protein (BMP). Targeting molecules preferably have an affinity for bone, such as tetracycline, calcein, bisphosphonate, polyaspartic acid, polyglutamic acid, aminophosphosugars, or estrogen.

It is another object of the invention to provide a method for augmenting bone formation in a subject.

It is another object of the invention to provide a method for treating bone loss in a subject.

### Modes of Carrying Out The Invention

### A. Definitions

The term "bone growth factor" refers to the families of proteins which affect bone formation. Examples of bone growth factors include transforming growth factor-beta (TGF-β), activin, and bone morphogenic factor (BMP).

The term "TGF-β" refers to beta-type transforming growth factors, including TGF-β1, TGF-β2, TGF-β3, TGF-β4, TGF-β5, heterodimers of the TGF-β polypeptide chains (e.g. TGF-β1.2), and fragments thereof, synthetic peptides, and homologous proteins having substantially equivalent biological activity in any of the numerous known TGF-β assays, such as the one described in Methods for Preparation of Media, Supplements, and Substrate for Serum-free Animal Cell Culture (1984) pp. 181-194. Alan R. Liss, Inc. This particular assay determines ability to induce anchorage-dependent growth in non-neoplastic normal rat kidney (NRK) fibroblasts by measuring the formation of cell colonies in soft agar. Other known TGF-β activity assays include but are not limited to stimulation of osteoblast proliferation, inhibition of keratinocyte proliferation, stimulation of collagen synthesis in a variety of cells, inhibition of mitogen-stimulated T-cell proliferation, and stimulation of chemotactic activity. Preparation of TGF-β1 and TGF-β2 is described in U.S. Patent No. 4,774,322. Additional TGF-βs have also been described. U.S. Patent No. 4,886,747 describes the identification of TGF-β3 and its nucleotide sequence, and describes a method for recovery of TGF-β from recombinant cell cultures. S. B. Jakowlew et al., Molec. Endocrinol. (1988) 2:1186-1195, describes TGF-β4 and its nucleotide sequence, identified by cDNA characterization. A.B. Roberts et al., Growth Factors, Vol. 2 (1990) pp. 135-147, describes the purification of TGF-β5 from Xenopus-conditioned medium.

The term "TGF-β" as used herein is also intended to include the heterodimer TGF-β2.3. TGF-β2.3 may be prepared from an extract of bone by pooling side fractions from peaks of column chromatography, subjecting those fractions to reverse phase HPLC and recovering those fraction which migrate more slowly than TGF-β2 by SDS-PAGE, subjecting those slower migrating fractions to FPLC and recovering those that migrate during a pH 4.6 to 6.7 gradient, subjecting the pH 4.6 to 6.7 eluant to reverse phase HPLC or gel electrophoresis, and recovering substantially pure TGF-β2.3.

The term "TGF-β" as used herein is also intended to include any other synthetic molecule whose mechanism of action is mediated through the TGF-β receptor or second messenger pathway.

Because these proteins are non-species-specific in their activity they maybe used to treat subjects in general, including sport, pet, and farm animals, and humans.

As used herein, "bone morphogenetic protein" (BMP) refers to a class of bone-derived proteins capable of inducing bone formation, as measured by activity in an in vivo rat bone formation assay. BMPs include BMP-1, BMP-2, BMP-3, BMP-4, BMP-5, BMP-6 and BMP-7, and fragments, deletions, additions, substitutions, mutations and modifications thereof which retain the biological characteristics of the natural BMP. The BMP may be of human, bovine, or other species origin.

The term "activin" as used herein refers to activin βAA, activin βAB, activin βBB, and fragments thereof, synthetic peptides, and proteins having similar activity in a standard cell culture assay where the protein stimulates the release of follicular stimulating hormone (FSH) from rat pituitary cells (Vale, et al., Nature (1986) 321:776). Briefly, in this assay, anterior pituitaries from adult male Sprague-Dawley rats (200-220 g) are dissociated by collagenase and plated at a concentration of 0.33 X 10⁶ cells per well in 24 well tissue culture dishes. The cells are allowed to recover for 72 hr in medium containing 2% fetal bovine serum (FBS). Following the recovery period, the cells are washed twice in fresh medium containing 2% FBS. All treatments are added at this time and the cells are incubated for 72 hr. The media is then collected and the FSH levels are determined using a radioimmunoassay kit provided by the National Hormone and Pituitary program of NIADDK.

As used herein, the term "targeting molecule" refers to a molecule that binds to the tissue of interest or that influences metabolism of that tissue of interest. So, for example, bone-targeting molecules may include bone-seeking molecules such as tetracycline, calcein, bisphosphonates, chelators, phosphates, polyaspartic acid, polyglutamic acid, aminophosphosugars, peptides known to be associated with the mineral phase of bone such as osteonectin, bone sialoprotein and osteopontin, proteins with bone mineral binding domains, and the like. Bone-targeting molecules may also include molecules which affect bone resorption and bone formation rates, such as bisphosphonates, and estrogens and other steroids. These bone-targeting molecules may bring the bone growth factors to the bone and/or result in a synergistic or additive effect on bone resorption or formation.

The term "hydrophilic polymer" as used herein refers to a synthetic or natural polymer having an average molecular weight and composition which renders the polymer essentially water-soluble. Most hydrophilic polymers achieve this property by incorporating a sufficient number of oxygen (or less frequently nitrogen) atoms available for forming hydrogen bonds in aqueous solution. Hydrophilic polymers used herein will generally be propylene glycol, polyoxyethylene, polyethylene glycol, polytrimethylene glycols, polylactic acid, or derivatives thereof. Other suitable polymers include polyoxyethylene-polyoxypropylene block polymers and copolymers. Naturally occurring polymers such as starch, heparin and the like are also included within the scope of this invention. All suitable polymers will be non-toxic and non-inflammatory when administered subcutaneously, and will preferably be essentially non-degradable in vivo over a period of several months. Presently preferred hydrophilic polymers are synthetic, preferably polyethylene glycols (PEG) having an average molecular weight between about 200 and about 10,000, more preferably between about 600 and about 8,000, and most preferably about 3400.

The PEG is preferably difunctional, i.e., a crosslinker between the bone growth factor and the targeting molecule, consisting of homobifunctional groups such as in PEG 600 diglycidylether, or of heterofunctional groups. Branched polyfunctional PEG crosslinkers may also be used. The nature of the functional groups on the crosslinker depends on the reactivities of the molecules to be conjugated.

The term "chemically conjugated" as used herein means attached through a covalent chemical bond. In the practice of the invention, a hydrophilic polymer and a bone growth factor may be chemically conjugated by using a linking radical, so that the polymer and the bone growth factor are each bound to the radical, but not directly to each other.

Those of ordinary skill in the art will appreciate that polymers such as polyethylene glycol cannot practically be prepared having exact molecular weights, and that the term "molecular weight" as used herein refers to the average molecular weight of a number of molecules in any given sample, as commonly used in the art. Thus, a sample of PEG 2000 might contain polymer molecules ranging in weight from, for example, 1,200 to 2,500 daltons. Specification of a molecular weight range indicates that the average molecular weight may be any value between the limits specified, and may include molecules outside those limits. Thus, a molecular weight range of about 800 to about 20,000 indicates an average molecular weight of at least about 800, ranging to about 20 kD.

The term "available lysine residue" as used herein refers to lysine side chains exposed on the outer surface of the bone growth factor or targeting molecule, which are positioned in a manner allowing reaction with activated PEG. In general, 10-100%, and more preferably, 10-50% of available lysine residues may be used. The number of available lysine residues may be determined by reaction with sodium 2,4,6-trinitrobenzenesulfonate (TNBS). Bone growth factor genetic variants can be constructed so as to substitute lysine residues at specific sites in the molecule, thus improving the efficiency of the crosslinking. In practice, such substitution will be conservative so as not to interfere with the receptor binding of growth factor and will be within exterior or more hydrophilic regions of the growth factor.

The term "treat" or "treatment" as used herein refer to repair, prevention, or alleviation of bone defects, especially defects due to loss of bone. Treatment of bone defects includes augmentation or restoration of lost bone as well as the prevention of further bone loss due to metabolic disease, chronic inflammatory processes (e.g., osteoporosis, osteoarthritis, Paget's disease, osteomalacia, osteohalisteresis, multiple myeloma and other forms of cancer, and age-related loss of bone mass) or steroid therapy. Compositions of the invention may include additional biologically active factors to aid in healing or regrowth of normal tissue.

The term "tissue of interest" as used herein refers to a desired target in the body for treatment or for placement of the bone growth factor. Tissues of interest may include bone, cartilage, or other tissues or cell types to which bone growth factors may be targeted.

### B. General Methods

The compositions of the invention comprise a bone growth factor chemically conjugated to a targeting molecule. Examples of chemistries for conjugating or coupling proteins have been summarized in the art (see, for example, P.Tyle and B. Ram, eds., Targeted Therapeutic Systems, Marcel Dekker, New York, 1990; and Means and Feeney, "Chemical Modifications of Proteins, History and Applications," Bioconjugate Chem. 1:2-12 (1990)).

One such method includes chemical conjugation of the bone growth factor to a selected synthetic hydrophilic polymer or polymers, which in turn is linked to a targeting molecule. Thus, for example, a reactive group on the bone growth factor may be conjugated through a crosslinker to a polymer, and the targeting molecule may be conjugated to the bone growth factor-polymer through a second crosslinker, where the second crosslinker may be of the same or different species as the first, depending on the reactive groups involved in the targeting molecule. Examples of reactive groups on proteins include but are not limited to the carboxyl groups of the C-terminus or of aspartic and glutamic acid; amino groups of N-terminal and lysine residues; imidazole of histidine and phenolic functions of tyrosine; sulfhydryl groups of cysteine; and guanidine groups of arginine.

For example, TGF-β contains a number of available amino, carboxyl, and hydroxy groups which may be used to bind the synthetic hydrophilic polymer. The polymer may be connected using a "linking group", as the native hydroxy or amino groups in TGF-β and in the polymer frequently require activation before they can be linked. One may thus employ compounds such as dicarboxylic anhydrides (e.g., glutaric or succinic anhydride) to form a polymer derivative (e.g., succinate), which may then be activated by esterification with a convenient leaving group, for example, N-hydroxysuccinimide, N,N'-disuccinimidyl oxalate, N,N'-disuccinimidyl carbonate, and the like. Presently preferred dicarboxylic anhydrides that are used to form polymer-glutarate compositions include glutaric anhydride, adipic anhydride, 1,8-naphthalene dicarboxylic anhydride, and 1,4,5,8-naphthalenetetracarboxylic dianhydride. The polymer thus activated is then allowed to react with the TGF-β, forming a TGF-β-polymer conjugate.

TGF-β is difficult to dissolve in solutions of appropriate pH for coupling to hydrophilic polymers. In a presently preferred method, the TGF-β is lyophilized in the absence of a carrier protein, and dissolved in a mild acid, preferably about 10 mM HCl. The solution is neutralized by adding a strong base, preferably NaOH (1 N solution) in buffered saline, additionally including a solubilizing amount of DMSO. The final solution preferably contains about 50% DMSO or CH₃CN to solubilize the TGF-β and to prevent aggregation.

In one embodiment, monomethylpolyethylene glycol (mPEG) (mw 5,000) is reacted with glutaric anhydride to form mPEG glutarate. The glutarate derivative is then reacted with N-hydroxysuccinimide to form a succinimidyl monomethylpolyethylene glycol glutarate. The succinimidyl ester is then capable of reacting with free amino groups present on a bone growth factor (lysine residues) to form a bone growth factor - PEG conjugate, which may then be further conjugated to a targeting molecule. Other polymers may be substituted for the mPEG, as described above. Similarly, the coupling reaction may be carried out using any known method for derivatizing proteins and synthetic polymers.

The activated mPEG may be replaced, in whole or in part, by bifunctional activated PEG (i.e., non-methylated PEG which is then activated at each end), thus providing a crosslinked or partially crosslinked bone growth factor composition. The character of the composition may be adjusted as desired, by varying the amount of bifunctional PEG included during the process.

The resulting bone growth factor-targeting molecule conjugates may be purified by standard techniques, preferably by reverse-phase HPLC (RP-HPLC), size-exclusion HPLC (SEC-HPLC; e.g., tetrahydrogel-HPLC) or ion-exchange chromatography. RP-HPLC is preferably performed using a C18 column using 90% acetonitrile or 90% isopropanol with 0.1% trifluoroacetic acid (TFA) as the B solvent. For SEC-HPLC, a preferred running buffer can be 5 mM sodium acetate at pH 5.5.

Whether a homobifunctional group or a heterobifunctional group is used depends on the nature of the reactive groups on the bone growth factor and the targeting molecule. For example, homobifunctional reagents may have the general formula R-PEG-R, where R is glycidylether (e.g., to form diglycidyl-PEG 600), succinimidyl succinate, ρ-nitrophenylcarbonate (e.g., to form bis(ρ-nitrophenylcarbonate) PEG3400, imidazoylcarbonyl, and the like. These and similar crosslinkers are available from Sigma, Union Carbide, and Polyscience. Shorter crosslinkers such as carbodiimide, not containing the PEG bridging part, have well described chemistries in the art and are available commercially (e.g., Pierce).

The bone growth factor-targeting compositions of the invention are preferably administered by parenteral routes, intravenous injection, intranasal or bronchial aerosol, and the like. Sustained release devices may be surgically implanted under the skin or within the peritoneal cavity.

Pharmaceutical formulations of the invention which include a bone growth factor and a targeting molecule for administration will generally include an osteogenically effective amount of the bone growth factor to promote bone growth, in addition to a pharmaceutically acceptable excipient. Suitable excipients include most carriers approved for parenteral administration, including water, saline, Ringer's solution, Hank's solution, and solutions of glucose, lactose, dextrose, ethanol, glycerol, albumin, and the like. These compositions may optionally include stabilizers, antioxidants, antimicrobials, preservatives, buffering agents, surfactants, and other accessory additives. The bone growth factor-targeting composition may also be delivered in a slow release form from a suitable carrier.

A presently preferred vehicle comprises about 1 mg/ml serum albumin (species-specific) in phosphate-buffered saline (PBS) or isotonic citrate buffer. A thorough discussion of suitable vehicles for parenteral administration may be found in E.W. Martin, "Remington's Pharmaceutical Sciences" (Mack Pub. Co., current edition sections relating to the excipient vehicles and formulating being incorporated herein by reference to disclose such). Such formulations are generally known to those skilled in the art and are administered systemically to provide systemic treatment.

The "effective amount" of bone growth factor - targeting composition necessary to effect treatment will depend upon the growth factor, the nature and severity of the condition to be treated, the age and general health of the subject, the specific activity of the composition prepared, and other factors which may be determined by the practitioner of ordinary skill in the art. As a general guide, however, an effective amount of bone growth factor-targeting molecule conjugate for treating a bone loss or defect will range from about 0.1 µg/kg to about 25 µg/kg.

The bone growth factor - targeting compositions of the invention may be formulated as solutions or suspensions, or may be lyophilized for later reconstitution.

### C. Examples

The examples presented below are provided as a further guide to the practitioner of ordinary skill in the art, and are not to be construed as limiting the invention in any way.

### Example 1

### (TGF-β-PEG-Tetracycline Conjugate Preparation)

Tetracycline (2 µmol; Aldrich Chem. Co.) is dissolved in 1 µmol bis-epoxy-PEG (Polyscience) and reacted at 90°C under nitrogen for 24 hr with gentle stirring. The resulting tetracycline-PEG conjugate is then reacted with TGF-β2 by adding 100 µg TGF-β2 dissolved in 0.02 M sodium borate, 0.02% SDS, 50% acetonitrile, pH 9.0 to 52 µl of the tetracycline-PEG conjugate. The mixture is incubated at 37° C for 48 hr. The resulting TGF-β-PEG-tetracycline conjugates are then purified by molecular sieve chromatography and C18-RP-HPLC.

### Example II

### (Conjugation of TGF-β2 to 3-amino-1-hydroxypropylidene-1,1-bisphosphonates (ADP) via bis-epoxy PEG 600).

Lyophilized TGF-β is dissolved in 50% water/acetonitrile (final-concentration 200-500 µg/ml). The pH is adjusted to 7-8 with phosphate or borate buffer. A 1-20 molar excess of bis-epoxy-PEG 600 or tetra-epoxy PEG 1700 (Polyscience) is added and the mixture is reacted for 15-20 hours at pH 7.0-8.0 at 40°C. Amicon ultrafiltration may be used to remove unattached PEG. The pH is raised to 9.5 with 1% deoxycholate, pH 9.5, 0.01 N NaOH, or 0.1 M borate buffer. The 3-amino 1-hydroxypropylidene 1,1-bisphosphonate (APD, CIBA-GEIGY) is dissolved in 1% deoxycholate, pH 9.5, at 2 mg/ml. The bisphosphonate is then added in a 5-200 molar excess and stirred overnight at 40°C. The reaction mixture is acidified and filtered through a 0.45 µm low protein binding syringe filter prior to chromatography. Purification is performed by gel filtration, ion exchange (Mono-S) and reverse-phase HPLC.

### Example III

### (Conjugation of TGF-β to 3-amino-1-hydroxypropylidene-1,1-bisphosphonate (APD) carbodiimide)

Lyophilized TGF-β (200 µg) is dissolved in 0.1 M MES (2-(N-Morpholino) ethanesulfonic acid) buffer, 50% acetonitrile, at pH 4.7. Sulfo-NHS (N-hydroxysulfosuccinimide, Pierce) is dissolved in aqueous 50% acetonitrile and 2-10 µmoles are added to the TGF-β solution. The water soluble 1-ethyl-3-(3-dimethylaminopropyl)carbodiimide HCL (EDC, Pierce) is dissolved in 0.1 MES buffer, pH 4.7, and added in 2-50 molar amounts. Finally, the bisphosphonate (ADP, CIBA-GEIGY) dissolved in 0.1 N HCl (5 mg/100 µl) is added in 2-50 µmolar amounts. The pH is adjusted to 4.7-5.5 with 0.01 N NaOH. The mixture is reacted overnight at room temperature. The TGF-β/APD conjugate is purified by ion-exchange (Mono-S) chromatography and reverse-phase HPLC.

### Example IV

### (Conjugation of TGF-β to Tetracycline (Einhorn Reaction))

Tetracycline dissolved in methanol is reacted with 38% formaldehyde. TGF-β dissolved in a 1:5 solution of water:methanol is added dropwise. The reaction mixture is stirred gently for 2 hr at room temperature. The resulting TGF-β-N-lysinemethyltetracycline can be separated from other reaction products by size-exclusion and RP-HPLC. (Bernardelli, et al. Liebigs Ann. Chem. 706:243-249 (1967)).

### Example V

### (Conjugation of TGF-β to Tetracycline)

Alkoxyalkyltetracyclines can be obtained by condensing a tetracycline with an aldehyde in alcohol. For example, tetracycline, mono-hydroxy-methoxy PEG-2000 (MeO-PEG 2000-OH), and formalin are added in equimolar amounts to a round bottom flask fitted with a reflux condenser. The mixture is dissolved in tert butyl alcohol by heating to approximately 80°C (oil bath). The temperature is increased to 115°C and held for 50 hours. This reaction is conducted under nitrogen. The system is then attached to a water aspirator to remove water from the reaction mixture, after which the mixture is allowed to cool to room temperature. Ethanol is added to dissolve the tert butyl alcohol and the mixture is filtered. The remaining wine-red precipitate is further purified by acetone extraction. The so-obtained intermediate can be further purified by ion exchange and size exclusion chromatography. After activation, the tetracycline-PEG conjugate can then be coupled to the TGF-β, preferably in an organic solvent such as methylene chloride, dimethylformamide, DMSO, and the like.

## Claims

1. A composition comprising a bone growth factor and a bone targeting molecule, wherein the bone growth factor and targeting molecule are chemically conjugated to each other via a crosslinker and the targeting molecule is not collagen.

2. A composition according to claim 1 wherein the bone growth factor is TGF-β.

3. A composition according to claim 1 wherein the bone growth factor is an activin.

4. A composition according to claim 1 wherein the bone growth factor is a bone morphogenic protein (BMP).

5. A composition according to any one of claims 1 to 4 wherein the crosslinker is polyfunctional.

6. A composition according to any one of claims 1 to 5 wherein the crosslinker is a synthetic hydrophilic polymer.

7. A composition according to claim 6 wherein the polymer is polyethylene glycol.

8. A composition according to claim 7 wherein the polyethylene glycol has a molecular weight of about 200 to about 10,000.

9. A composition according to claim 6 wherein the synthetic hydrophilic polymer is bound to an available lysine residue on the bone growth factor.

10. A composition according to claim 6 wherein the growth factor has a plurality of available lysine residues and molecules of the synthetic hydrophilic polymer are bound to 10 to 100% of the available lysine residues.

11. A composition according to claim 6 wherein the growth factor has a plurality of available lysine residues and molecules of the synthetic hydrophilic polymer are bound to 10 to 50% of the available lysine residues.

12. A composition according to any preceding claim wherein the targeting molecule is a protein.

13. A composition according to any one of claims 1 to 11 wherein the targeting molecule is tetracycline.

14. A composition according to any one of claims 1 to 11 wherein the targeting molecule is calcein.

15. A composition according to any one of claims 1 to 11 wherein the targeting molecule is a bisphosphonate.

16. A composition according to any one of claims 1 to 11 wherein the targeting molecule is polyaspartic acid.

17. A composition according to any one of claims 1 to 11 wherein the targeting molecule is polyglutamic acid.

18. A composition according to any one of claims 1 to 11 wherein the targeting molecule is an aminophosphosugar.

19. A composition according to any one of claims 1 to 11 wherein the targeting molecule is a steroid.

20. A composition according to any one of claims 1 to 11 wherein the targeting molecule is an estrogen.

21. A composition according to any preceding claim which contains a sustained-release vehicle.

22. A composition according to any preceding claim comprising a pharmaceutically acceptable carrier.

23. A composition according to any preceding claim for use in a method of treating the human or animal body for augmenting the formation of mature bone.

24. A composition according to claim 22 for use in a method for treating bone loss in a human or animal body, wherein the bone loss is osteoporosis-related or osteomalacia-related or osteohalisteresis-related or age-related or is related to steroid therapy or is osteoarthritis-related or is related to Paget's disease or is related to cancer, e.g. multiple myeloma or wherein the treatment is prophylactic.

25. Use of a composition according to any of claims 1 to 22 for the manufacture of a medicament for treating bone loss or augmenting the formation of mature bone.

## Patentansprüche

1. Zusammensetzung, umfassend einen Knochenwachstumsfaktor und ein Knochenzielmolekül, wobei der Knochenwachstumsfaktor und Zielmolekül über ein Vernetzungsmittel chemisch miteinander verknüpft sind und das Zielmolekül nicht Kollagen ist.

2. Zusammensetzung nach Anspruch 1, wobei der Knochenwachstumsfaktor TGF-β ist.

3. Zusammensetzung nach Anspruch 1, wobei der Knochenwachstumsfaktor ein Aktivin ist.

4. Zusammensetzung nach Anspruch 1, wobei der Knochenwachstumsfaktor ein knochenmorphogenetisches Protein (BMP) ist.

5. Zusammensetzung nach einem der Ansprüche 1 bis 4, wobei das Vernetzungsmittel polyfunktionell ist.

6. Zusammensetzung nach einem der Ansprüche 1 bis 5, wobei das Vernetzungsmittel ein synthetisches hydrophiles Polymer ist.

7. Zusammensetzung nach Anspruch 6, wobei das Polymer Polyethylenglykol ist.

8. Zusammensetzung nach Anspruch 7, wobei das Polyethylenglykol ein Molekulargewicht von etwa 200 bis etwa 10.000 hat.

9. Zusammensetzung nach Anspruch 6, wobei das synthetische hydrophile Polymer an einen verfügbaren Lysinrest am Knochenwachstumsfaktor gebunden ist.

10. Zusammensetzung nach Anspruch 6, wobei der Wachstumsfaktor eine Vielzahl von verfügbaren Lysinresten hat und Moleküle des synthetischen hydrophilen Polymers an 10 bis 100% der verfügbaren Lysinreste gebunden sind.

11. Zusammensetzung nach Anspruch 6, wobei der Wachstumsfaktor eine Vielzahl von verfügbaren Lysinresten hat und Moleküle des synthetischen hydrophilen Polymers an 10 bis 50% der verfügbaren Lysinreste gebunden sind.

12. Zusammensetzung nach einem beliebigen vorstehenden Anspruch, wobei das Zielmolekül ein Protein ist.

13. Zusammensetzung nach einem der Ansprüche 1 bis 11, wobei das Zielmolekül Tetracyclin ist.

14. Zusammensetzung nach einem der Ansprüche 1 bis 11, wobei das Zielmolekül Calcein ist.

15. Zusammensetzung nach einem der Ansprüche 1 bis 11, wobei das Zielmolekül ein Bisphosphonat ist.

16. Zusammensetzung nach einem der Ansprüche 1 bis 11, wobei das Zielmolekül Polyasparaginsäure ist.

17. Zusammensetzung nach einem der Ansprüche 1 bis 11, wobei das Zielmolekül Polyglutaminsäure ist.

18. Zusammensetzung nach einem der Ansprüche 1 bis 11, wobei das Zielmolekül ein Aminophosphozucker ist.

19. Zusammensetzung nach einem der Ansprüche 1 bis 11, wobei das Zielmolekül ein Steroid ist.

20. Zusammensetzung nach einem der Ansprüche 1 bis 11, wobei das Zielmolekül ein Östrogen ist.

21. Zusammensetzung nach einem beliebigen vorstehenden Anspruch, die einen Träger für anhaltende Abgabe enthält.

22. Zusammensetzung nach einem beliebigen vorstehenden Anspruch, umfassend einen pharmazeutisch verträglichen Träger.

23. Zusammensetzung nach einem beliebigen vorstehenden Anspruch zur Verwendung in einem Verfahren zur Behandlung des menschlichen oder tierischen Körpers, um die Erzeugung von ausgereiftem Knochen zu vermehren.

24. Zusammensetzung nach Anspruch 22 zur Verwendung in einem Verfahren zur Behandlung von Knochenverlust in einem menschlichen oder tierischen Körper,
wobei der Knochenverlust verbunden ist mit Osteoporose oder Osteomalazie oder Osteohalisterese oder altersbedingt ist oder verbunden ist mit Steroidtherapie oder Osteoarthritis oder mit der Paget-Krankheit oder Krebs, z. B. multiplem Myelom, oder
wobei die Behandlung prophylaktisch ist.

25. Verwendung einer Zusammensetzung nach einem der Ansprüche 1 bis 22 zur Herstellung eines Arzneimittels zur Behandlung von Knochenverlust oder Unterstützung der Bildung von ausgereiftem Knochen.

## Revendications

1. Composition comprenant un facteur de croissance osseuse et une molécule de ciblage de l'os, dans laquelle le facteur de croissance osseuse et la molécule de ciblage sont conjugués chimiquement l'un à l'autre par l'intermédiaire d'un agent de réticulation, la molécule de ciblage n'étant pas du collagène.

2. Composition selon la revendication 1 dans laquelle le facteur de croissance osseuse est TGF-β.

3. Composition selon la revendication 1 dans laquelle le facteur de croissance osseuse est une activine.

4. Composition selon la revendication 1 dans laquelle le facteur de croissance osseuse est une protéine morphogénique de l'os (BMP).

5. Composition selon l'une quelconque des revendications 1 à 4 dans laquelle l'agent de réticulation est polyfonctionnel.

6. Composition selon l'une quelconque des revendications 1 à 5 dans laquelle l'agent de réticulation est un polymère hydrophile synthétique.

7. Composition selon la revendication 6 dans laquelle le polymère est du polyéthylène glycol.

8. Composition selon la revendication 7 dans laquelle le polyéthylène glycol a un poids moléculaire d'environ 200 à 10 000.

9. Composition selon la revendication 6 dans laquelle le polymère hydrophile synthétique est lié à un résidu de lysine disponible sur le facteur de croissance osseuse.

10. Composition selon la revendication 6 dans laquelle le facteur de croissance a une pluralité de résidus de lysine disponibles et les molécules du polymère hydrophile synthétique sont liées de 10 à 100 % aux résidus de lysine disponibles.

11. Composition selon la revendication 6 dans laquelle le facteur de croissance a une pluralité de résidus de lysine disponibles et les molécules du polymère hydrophile synthétique sont liées de 10 à 50 % aux résidus de lysine disponibles.

12. Composition selon l'une quelconque des revendications précédentes dans laquelle la molécule de ciblage est une protéine.

13. Composition selon l'une quelconque des revendications 1 à 11 dans laquelle la molécule de ciblage est une tétracycline.

14. Composition selon l'une quelconque des revendications 1 à 11 dans laquelle la molécule de ciblage est de la calcéine.

15. Composition selon l'une quelconque des revendications 1 à 11 dans laquelle la molécule de ciblage est un biphosphonate.

16. Composition selon l'une quelconque des revendications 1 à 11 dans laquelle la molécule de ciblage est de l'acide polyaspartique.

17. Composition selon l'une quelconque des revendications 1 à 11 dans laquelle la molécule de ciblage est de l'acide polyglutamique.

18. Composition selon l'une quelconque des revendications 1 à 11 dans laquelle la molécule de ciblage est un sucre aminophosphoré.

19. Composition selon l'une quelconque des revendications 1 à 11 dans laquelle la molécule de ciblage est un stéroïde.

20. Composition selon l'une quelconque des revendications 1 à 11 dans laquelle la molécule de ciblage est un oestrogène.

21. Composition selon l'une quelconque des revendications précédentes qui contient un véhicule à libération continue.

22. Composition selon l'une quelconque des revendications précédentes comprenant un excipient pharmaceutiquement acceptable.

23. Composition selon l'une quelconque des revendications précédentes pour une utilisation dans une méthode de traitement du corps humain ou animal pour augmenter la formation d'os mature.

24. Composition selon la revendication 22 pour une utilisation dans une méthode de traitement d'une perte osseuse dans un corps humain ou animal, dans laquelle la perte osseuse est liée à l'ostéoporose ou est liée à l'ostéomalacie ou est liée à l'ostéohalistérèse ou est liée à l'âge ou est liée à une thérapie par stéroïde ou est liée à l'arthrose ou est liée à la maladie de Paget ou est liée à un cancer, par exemple un myélome multiple ou dans une méthode de traitement prophylactique.

25. Utilisation d'une composition selon l'une quelconque des revendications 1 à 22 pour la préparation d'un médicament destiné à traiter une perte osseuse ou à augmenter la formation d'os mature.
